# EUROPÄISCHE PATENTSCHRIFT

(11) **EP 2 658 532 B1**
(45) Veröffentlichungstag und Bekanntmachung des Hinweises auf die Patenterteilung: **11.12.2019**
(21) Anmeldenummer: 11805542.5
(22) Anmeldetag: 29.12.2011
(51) Int. Cl.: A61K 31/15, A61K 31/4535, A61K 31/4468, A61K 31/485, A61P 25/04, A61K 9/70

(54) **TRANSDERMALES APPLIKATIONSSYSTEM MIT ÜBERSTEHENDER BACKINGFOLIE**
TRANSDERMAL APPLICATION SYSTEM COMPRISING A PROTRUDING BACKING FILM
SYSTÈME D'ADMINISTRATION TRANSDERMIQUE COMPRENANT UN FILM SUPPORT DÉBORDANT

(30) Priorität: 29.12.2010 DE 102010064358
(43) Veröffentlichungstag der Anmeldung: 06.11.2013
(73) Patentinhaber: Luye Pharma AG, 83714 Miesbach (DE)
(72) Erfinder: SALMAN, Nouha, 81373 München (DE); SCHURAD, Björn, 81667 München (DE); SCHNEIDER, Lorena, 83043 Bad Aibling (DE); BECKERT, Thomas, 88447 Birkenhard (DE)
(74) Vertreter: Zehetner Patentanwälte
(86) Internationale Anmeldenummer: PCT/EP2011/074236
(87) Internationale Veröffentlichungsnummer: WO 2012/089811

(56) Entgegenhaltungen:
- EP-A1- 1 062 926
- WO-A1-92/17237
- WO-A2-2010/042152
- US-A- 5 662 926

## Beschreibung

Die vorliegende Erfindung betrifft ein langzeitlagerfähiges transdermales Applikationssystem und insbesondere ein transdermales Applikationssystem mit einem effektiven Einschluss des bzw. der Wirkstoffe in der Matrix des Systems auch während längerer Lagerungszeiten.

Transdermale Applikationssysteme (Akronym TDS von englisch Transdermal Delivery System) dienen dem Verabreichen von Arzneistoffen durch die Haut eines Patienten in dessen Blutkreislauf. Sie umfassen gewöhnlich ein Reservoir für den Wirkstoff, eine wirkstoffundurchlässige Rückschicht sowie eine Schutzfolie, die vor einer Applikation des Systems entfernt wird.

Grundsätzlich können zwei verschiedene Arten von Systemen unterschieden werden, nämlich die so genannten Matrixsysteme, bei denen der Wirkstoff in einer Polymermatrix enthalten ist, die zumeist aus einem selbsthaftenden druckempfindlichen Polymer gebildet ist, und die so genannten Reservoirsysteme, die aus einem den Wirkstoff enthaltenden flüssigen, halbfesten oder festen Reservoir und einer die Wirkstoffabgabe regulierenden Membran bestehen.

Matrixsysteme enthalten den Wirkstoff oder die Wirkstoffformulierung in einer, z.B. als Klebstoffschicht ausgebildeten, nichtflüssigen Polymerschicht. An der der Haut abgewandeten Seite ist die wirkstoffhaltige Matrix mit einer Rückschicht versehen, während auf der der Haut zugewandeten Seite eine Schutzfolie die Matrix vor Verschmutzungen schützt, wodurch ein unkontrolliertes Austreten von Wirkstoff während einer Aufbewahrung des TDS verhindert wird.

In der Patentanmeldung DE 36 29 304 A1 ist ein therapeutisches System mit Wirkstoffdepot beschrieben worden, wobei neben einer Reservoirmatrix ein oder mehrere Wirkstoffdepots mit einer höheren Wirkstoffkonzentration als in der Reservoirmatrix enthalten sind. Während der Lagerung reichert sich der Wirkstoff in der Reservoirmatrix an, sodass durch die Abscheidung als Depot auch thermisch instabile oder flüchtige Wirkstoffe ohne thermische Belastung in das System eingebracht werden können. Das System kann neben einer ablösbaren Schutzschicht auch eine Rückschicht aufweisen, die mit einer Haftklebeschicht versehen ist.

Bei transdermalen Applikationssystemen erfolgt der Wirkstoffeintrag in die Haut eines Patienten durch Diffusion der Wirkstoffe entlang des Wirkstoffkonzentrationsgefälles, das zwischen der Matrix des TDS und dem vom Blutgefäßsystem durchsetzten Bereich der Haut besteht. Der mit einem transdermalen Applikationssystem erzielbare Wirkstoffblutspiegel wird, abgesehen von der Auflagefläche der Matrix auf der Haut des Patienten, im Wesentlichen über die Konzentration des Wirkstoffs in der Matrix bestimmt. Da die diffusionsregulierte Abgabe von Wirkstoff aus einem TDS zu einer Verringerung der Wirkstoffkonzentration in der Matrix führt, muss die anfängliche Wirkstoffkonzentration in der Matrix entsprechend hoch gewählt sein. Um der damit verbundenen Gefahr einer ungewollten Kontamination von mit dem TDS in Berührung kommenden Personen oder Gegenständen vorzubeugen, ist die Matrix bei einem TDS üblicherweise zwischen einer wirkstoffundurchlässigen Rückschicht und einer ebenfalls wirkstoffundurchlässigen abziehbaren Schutzfolie angeordnet.

Transdermale therapeutische Systeme können ferner mit einer als Overtape bezeichneten Deckschicht ausgestattet sein, insbesondere wenn die den Wirkstoff enthaltende Polymermatrix nicht oder nicht in ausreichendem Maße haftklebend ist, um ein sicheres Anbringen des TDS auf der Haut zu ermöglichen und/oder ein vorzeitiges Ablösen des TDS zu verhindern. Bei einem Overtape handelt es sich um ein mit einer wirkstofffreien Haftkleberschicht versehenes folienförmiges Material, das gewöhnlich wirkstoffundurchlässig ausgebildet oder mit einer wirkstoffundurchlässigen Rückschicht versehen ist. Das Overtape wird auf der der Haut abgewandeten Seite des TDS über dem Laminat aus wirkstoffhaltiger Matrix und wirkstoffundurchlässiger Rückschicht angebracht und überragt dieses Laminat in lateraler Richtung, sodass der überstehende Klebstoffrand zur Befestigung auf der Haut dienen kann. Nach Abziehen der Schutzfolie wird das TDS mit der nicht von der Matrix abgedeckten Kleberschicht der Deckschicht auf der Haut eines Patienten befestigt. Transdermale therapeutische Pflaster mit Klebstoffrand und ihr Herstellungsverfahren sind beispielsweise in dem Patent EP 577 622 beschrieben worden.

Es hat sich gezeigt, dass über die Randzonen der Matrix Wirkstoff austreten und in die Umgebung gelangen kann. Ferner weisen die für einige Wirkstoffe verwendeten Matrices kaltfließende Eigenschaften auf, wodurch die Matrices auch ohne besondere mechanische oder thermische Belastung zu Fließen beginnen und sich nach einiger Zeit in Bereiche außerhalb ihrer ursprünglichen Randzonen und damit auch in Bereiche außerhalb des von der wirkstoffundurchlässigen Rückschicht abgedeckten Bereichs hinaus erstrecken. In beiden Fällen entsteht dadurch eine erhebliche Kontaminationsgefahr für Dritte, sowohl beim Verabreichen als auch beim Tragen eines solchen TDS.

Zur Lösung der bei Verwendung von Matrices mit kaltfließenden Eigenschaften auftretenden Probleme wird in der Patentanmeldung EP 1 062 926 A1 ein flächiges selbstklebendes Pflaster vorgeschlagen, bei dem ein Klebstoffkern aus einem fließfähigen Klebstoff von einem Klebstoffring mit herabgesetzter Fließfähigkeit umgeben ist, der den Klebstoff der inneren Schicht mechanisch am Ausfließen hindert.

Im Kontext der vorliegenden Erfindung wird als "kaltfließende Matrix" eine Matrix bezeichnet, die bei Raumtemperatur eine extrem langsame Fließfähigkeit aufweist. Insbesondere ist eine "kaltfließende Matrix" eine Matrix, die als solche eine Fließfähigkeit aufweist, die von bloßem Auge erst nach einer mindestens 6-monatigen Lagerung bei 25°C erkennbar ist.

Wirkstoffe, die über die Randbereiche der Matrix oder aufgrund eines Verfließens der Matrix aus über die Rückschicht hinausreichenden Bereichen der Matrix ausgetreten sind, können zudem in die Deckschicht gelangen und sich dort anreichern. Bei einer Lagertemperatur von 25 °C können so nach 17 Monaten bereits mehr als 5% und nach 30 Monaten bereits mehr als 8% des Wirkstoffs von der Matrix in die Deckschicht übergetreten sein. Abgesehen von der damit verbundenen Kontaminationsgefahr nimmt der nutzbare Wirkstoffgehalt durch den damit verbundenen Wirkstoffverlust in der Matrix im Laufe der Zeit ab, so dass nach einer längeren Lagerung des TDS die geforderte Konstanz des Wirkstoffgehalts in der Matrix und in der Folge die gewünschten Wirkstoffblutspiegel nicht mehr erreicht werden können.

Es ist daher wünschenswert, ein transdermales Applikationssystem so auszubilden, dass einem Austritt der Matrix sowie der darin enthaltenen Wirkstoffe aus dem von einer wirkstoffundurchlässigen Rückschicht begrenzten Bereich sowohl während der Lagerung als auch während der Anwendung vorgebeugt ist.

Im Kontext der vorliegenden Erfindung bedeutet "lagerstabiles, transdermales Applikationssystem", dass beim Betrachten des gebrauchsfertigen transdermalen Applikationssystems nach einer 6-monatigen Lagerung bei 25°C von bloßem Auge kein kalter Fluss erkennbar ist und/oder dass der Wirkstoffgehalt der wirkstoffhaltigen Matrix des gebrauchsfertigen transdermalen Applikationssystems nach 17 Monaten bei einer Lagertemperatur von 25 °C um weniger als 5%, vorzugsweise um weniger als 2% und am bevorzugtesten um weniger als 1% abgenommen hat und/oder dass der Wirkstoffgehalt der wirkstoffhaltigen Matrix des gebrauchsfertigen transdermalen Applikationssystems nach 30 Monaten bei einer Lagertemperatur von 25 °C um weniger als 8%, vorzugsweise um weniger als 5% und am bevorzugtesten um weniger als 2% abgenommen hat.

Die Erfindung ist in den unabhängigen Ansprüchen definiert. Die Unteransprüche beziehen sich auf bevorzugte Ausführungsformen.

Erfindungsgemäße transdermalen Applikationssystemen mit einer Begrenzung des Austritts von Matrix und/oder Wirkstoff aus dem von einer wirkstoffundurchlässigen Rückschicht abgedeckten Bereich weisen eine zwischen einer wirkstoffundurchlässigen Rückschicht und einer abziehbaren Schutzfolie angeordnete wirkstoffhaltige Matrix auf, wobei die Rückschicht so ausgebildet ist, dass sie die Ränder der Matrix überlappt.

Unter Überlappung oder Überstand der Rückschicht ist im Rahmen dieser Schrift zu verstehen, dass die laterale Ausdehnung der Rückschicht in x- und y-Richtung, d.h. die nach dem Aufbringen des TDS in etwa parallel zur Haut verlaufende Ausdehnung, immer größer ist als die Ausdehnung der Matrix in x- und y-Richtung. Die vertikale z-Richtung verläuft demgemäß in der Richtung der Dicke des Pflasters in etwa senkrecht zur Hautoberfläche. Bevorzugt ist die Überlappung gleichförmig, d.h. sie weist an allen Rändern der Matrix in etwa den gleichen Wert auf.

Das durch die Überlappung erzielte Abdecken der Matrixränder durch die wirkstoffundurchlässige Rückschicht beugt einem ungewollten Kontaktieren des Matrixrands durch z.B. weitere Komponenten des transdermalen Applikationssystems, beispielsweise durch eine wie oben beschriebene Deckschicht, durch die Verpackung des TDS oder durch Personen wirksam vor. Mithilfe der Überlappung kann auch ein Verfließen kaltfließender Matrices über den Rand der Rückschicht hinaus wirksam unterbunden werden.

Die transdermalen Applikationssystemen mit einer Begrenzung eines Austritts von Matrix und/oder von Wirkstoff aus dem von einer wirkstoffundurchlässigen Rückschicht abgedeckten Bereich weisen einen Überstand der Ränder der Rückschicht über die Ränder der Matrix auf, der wenigstens dem Abstand zwischen der Rückschicht und der abziehbaren Schutzfolie am Rand der Matrix entspricht. Dies ermöglicht ein Umklappen der überstehenden Rückschichtbereiche so, dass diese die Seitenränder der Matrix vollständig nach außen abdecken und einer ungewollten Kontamination der Matrixumgebung, und hierüber von Personen bei der Handhabung des Systems, noch wirksamer vorgebeugt werden kann.

Die erfindungsgemäßen transdermalen Applikationssysteme weisen einen Überstand der Ränder der Rückschicht über die Ränder der Matrix auf, dessen Wert aus dem Bereich des Zweifachen bis Zehnfachen des Abstands zwischen der Rückschicht und der abziehbaren Schutzfolie am Rand der Matrix, vorzugsweise aus dem Bereich des Zweifachen bis Fünffachen und besonders bevorzugt aus dem Bereich des Zweifachen bis Dreifachen ausgewählt ist.

Der Randbereich der Rückschicht, der mit der in der Fachsprache auch als Releaseliner bezeichneten abziehbaren Schutzfolie in Kontakt ist, ist haftklebend ausgeführt. Bei der Aufbewahrung des TDS wird hierdurch der Luftzutritt zu den Randbereichen der Matrix wirksam unterbunden, wodurch einer Zersetzung von in der Matrix enthaltenen Wirkstoffen und insbesondere von sauerstoffempfindlichen Wirkstoffen vorgebeugt und die Langzeitstabilität des Pflasters verbessert werden kann.

Bevorzugte Ausführungsformen solcher transdermaler Applikationssysteme weisen ferner eine Deckschicht auf, die die Rückschicht vollständig überdeckt und mit der abziehbaren Schutzfolie außerhalb der Ränder der Rückschicht haftklebend verbunden ist. Ein solcherart ausgebildetes TDS ermöglicht eine einfache Applikation auf der Haut und verringert zudem die Gefahr einer Berührung der Matrix während des Aufbringens. Die Deckschicht ermöglicht zudem ein in weiten Bereichen von den Klebeeigenschaften der Matrix unabhängiges Anbringen des TDS auf der Haut einer Person oder eines Tieres. Bei sehr fließfähigen Matrices kann außerdem der kalte Fluss eingedämmt werden, wobei die vorhandene überstehende Rückschicht einen Übertritt von Wirkstoff aus der Matrix in die wirkstofffreie Kleberschicht der Deckschicht verhindert.

Bei den mit einer Deckschicht versehenen Ausführungsformen entspricht der Überstand der Deckschicht über die Ränder der Rückschicht wenigstens dem Dreifachen des Abstands zwischen Deckschicht und abziehbarer Schutzfolie am Rand der Matrix.

Vorteilhafte Ausführungsformen der oben beschrieben transdermalen Applikationssysteme weisen ferner eine Matrix-Randumfassung auf, die an die Ränder der Matrix angrenzt und von der wirkstoffundurchlässigen Rückschicht überdeckt ist. Die Matrix-Randumfassung wirkt als Randstütze, die die Matrix stabilisiert und insbesondere bei stark kaltfließenden Matrices ein Verfließen der Matrixränder verhindert.

Weitere Merkmale der Erfindung ergeben sich aus der nachfolgenden Beschreibung von Ausführungsbeispielen in Verbindung mit den Ansprüchen sowie den Figuren. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung einiger Ausführungsbeispiele der Erfindung wird auf die beiliegenden Figuren Bezug genommen, von denen
- Figur 1a: eine schematisierte Darstellung eines Querschnitts durch ein transdermales Applikationssystem mit einer Begrenzung des Austritts von Matrix und/oder Wirkstoff aus dem von einer wirkstoffundurchlässigen Rückschicht abgedeckten Bereich zeigt,
- Figur 1b: in einer schematischen Darstellung das Applikationssystem von Fig. 1a in einer Draufsicht zeigt,
- Figur 2: zwei Modifikationen der in Figur 1 veranschaulichten Ausführungsform eines transdermalen Applikationssystems zeigt,
- Figur 3: verschiedene Ausgestaltungen eines transdermalen Applikationssystems veranschaulicht und
- Figur 4: eine schematisierte Darstellung eines Querschnitts durch ein transdermales Applikationssystem mit einer Begrenzung des Austritts von Matrix und/oder Wirkstoff aus dem von einer wirkstoffundurchlässigen Rückschicht abgedeckten Bereich gemäß einer weiteren Ausführungsform zeigt.

In den Figuren sind Elemente die im Wesentlichen gleiche technische Funktionen erfüllen mit gleichen Bezugszeichen versehen. Verschieden Ausgestaltungen dieser Elemente weisen ähnliche Bezugszeichen auf.

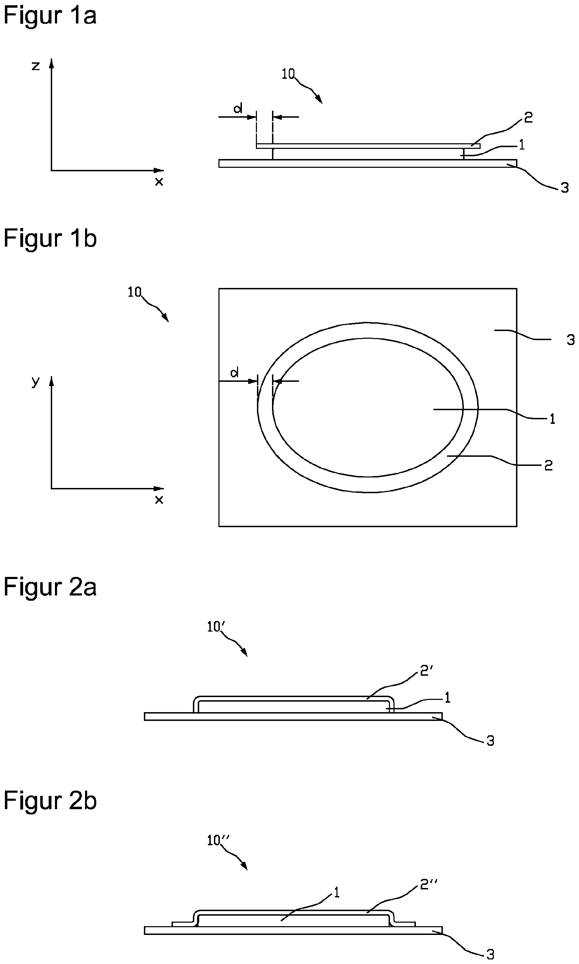

Figur 1a zeigt einen schematisierten Querschnitt durch ein transdermales Applikationssystem 10. In der schematischen Darstellung von Fig. 1b ist dieses System i in Draufsicht gezeigt. Das System 10 weist eine wirkstoffhaltige Matrix 1 auf, die auf der zum Kontakt mit der Haut vorgesehenen Seite von einer abziehbaren Schutzfolie 3 und auf der anderen Seite von einer wirkstoffundurchlässigen Rückschicht 2 abgedeckt ist. Die zwischen Rückschicht 2 und Releaseliner 3 angeordneten Seitenflächen der Matrix 1 begrenzen die Ausdehnung der Matrix 1 in x- und y-Richtung und werden als Ränder der Matrix bezeichnet. Die Rückschicht 2 überragt die Matrixränder in x- und y-Richtung, in der dargestellten Ausführungsform um eine Distanz d. Dieser auch als Überlappung bezeichnete Überstand kann an allen Bereichen der Matrixränder konstant sein, kann an unterschiedlichen Bereichen der Matrixränder jedoch auch unterschiedliche Werte annehmen. In der in Fig. 1a und 1b gezeigten Ausführungsform sind sowohl die Matrix als auch die Rückschicht oval ausgebildet und die Überlappung d ist in allen Bereichen konstant. Natürlich sind für die Applikationssysteme grundsätzlich auch andere Formen möglich, wie z.B. runde, quadratische oder rechteckförmige Formen, gegebenenfalls mit abgerundeten Ecken, oder beliebige andere geeignete Formen.

Die Matrix eines transdermalen Applikationssystems weist üblicherweise eine Dicke im Bereich von 30 bis 500 µm auf, so dass nur durch den Überstand der wirkstoffundurchlässigen Rückschicht 2 über die Ränder der Matrix 1 ein wirksamer Schutz gegenüber einer ungewollten Kontaktierung der Matrixränder durch eine Person oder durch Verpackungsmaterialien gebildet wird.

Eine modifizierte Ausführung des in Figur 1 veranschaulichten transdermalen Applikationssystems ist in Figur 2a dargestellt. Als Rückschichten 2 werden üblicherweise sogenannte Backingfolien aus Polyester mit einer Stärke von maximal 15 µm und vorzugsweise aus dem Bereich von 6 bis 12 µm verwendet. Solche Backingfolien sind hochflexibel und können sich, wie in Figur 2a dargestellt, um die Ränder der Matrix 1, d.h. um die in lateralere Richtungen weisenden Seitenflächen der Matrix 1 legen und diese abdecken. Bevorzugt basiert die Rückschicht auf einem Polymer ausgewählt aus der Gruppe bestehend aus Polyolefinen, Olefin-Copolymerisaten, Polyestern, CoPolyestern, Polyamiden, Co-Polyamiden, Polyurethanen, und dergleichen mehr. Als Beispiele für geeignete Materialien können Polyester und hiervon insbesondere Polyethylenterephthalate als auch Polycarbonate genannt werden, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Copolymerisate wie beispielsweise Acrylnitril-Butadien-Styrol-Terpolymere oder Ethylen-Vinylacetat-Copolymerisate.

In der dargestellten Modifikation 10' entspricht der Überstand der Ränder der Rückschicht 2 über die Ränder der Matrix 1 dem Abstand zwischen der Rückschicht 2 und der abziehbaren Schutzfolie 3 am Rand der Matrix 1. Die Rückschicht 2 überlappt die Ränder der Matrix in diesem Fall um einen Wert, der der Höhe der Matrixränder entspricht, wodurch der Überstand der Rückschicht 2 die Ränder der Matrix 1 vollständig abdeckt und so einem Austreten von Wirkstoff aus der Matrix oder einem Verfließen der Matrix wirksam vorbeugt.

In Figur 2b ist eine weitere modifizierte Ausgestaltung 10" des in Figur 1 veranschaulichten transdermalen Applikationssystems dargestellt. Im Vergleich zu der in Figur 2a veranschaulichten Ausgestaltung 10" ist der Überstand der Rückschicht 2 über die Matrixränder größer als der am Matrixrand vorliegende Abstand zwischen Rückschicht 2 und abziehbarer Schutzfolie 3 ausgeführt. Hierdurch deckt die Rückschicht 2 nicht nur die Matrixränder ab, sondern liegt außerhalb der Matrixränder flächig auf der Schutzfolie auf.

Für die Matrix eignen sich grundsätzlich alle Polymere, die bei der Herstellung von transdermalen Systemen eingesetzt werden und physiologisch unbedenklich sind. Zu nennen sind beispielsweise natürlicher Kautschuk, synthetischer Kautschuk, Homo- und Copolymere auf Acrylbasis, Polyvinylacetat, Polyisobutylen, Silicone, insbesondere Polydimethylsiloxan, und Hydrogele wie z. B. Polyvinylpyrrolidon, Polyvinylalkohol und Polyethylenoxid-Polymere.

Bei einer weiteren, in den Figuren nicht dargestellten Modifikation dieser Ausgestaltung ist zumindest die auf der Schutzfolie 3 außerhalb der Matrixränder aufliegende Fläche der Rückschicht 2 mit einer haftklebenden Beschichtung versehen. Diese lösbare Klebeverbindung schließt die Matrix 1 sicher in dem zwischen der Rückschicht 2 und der abziehbaren Schutzfolie 3 gebildeten Raum ein und beugt somit nicht nur einem Austreten von z.B. toxischen Wirkstoffen oder auch der Matrix selbst aus dem Verbund vor, sondern unterbindet auch den Luftzutritt zur Matrix, wodurch sich die Lagerfähigkeit eines transdermalen Applikationssystems mit luftempfindlichen Wirkstoffen deutlich erhöht. Besonders vorteilhaft ist diese Ausführungsform für Systeme, die einen Wirkstoff enthalten, der keinesfalls unkontrolliert in die Umgebung gelangen soll. Beispiele hierfür sind etwa die als Analgetika dienenden Opioide Fentanyl, Buprenorphin, Remifentanil sowie Sulfentanil, die dem Betäubungsmittelgesetz unterliegen und besonders heikel zu handhaben sind.

Der Überstand der Ränder der Rückschicht über die Ränder der Matrix weist hierfür erfindungsgemäß bei einer ersten Ausführungsform einen Wert aus dem Bereich des zwei- bis zehnfachen des Abstands der Rückschicht von der Schutzfolie am Rand der Matrix auf. Insbesondere bei Verwendung dünner Backingfolien für die Ausbildung der Rückschicht 2 weist die Überlappung der Rückschicht vorzugsweise Werte aus dem Bereich des zwei- bis fünffachen und besonders bevorzugt aus dem Bereich des zwei- bis dreifachen des Abstands der Rückschicht von der Schutzfolie am Rand der Matrix auf. Selbstverständlich können auch wesentlich größere Überstände verwendet werden, insbesondere dann, wenn der mit einem Haftkleber versehene Überlappungsrand auch zur besseren Fixierung des TDS auf der Haut verwendet werden soll.

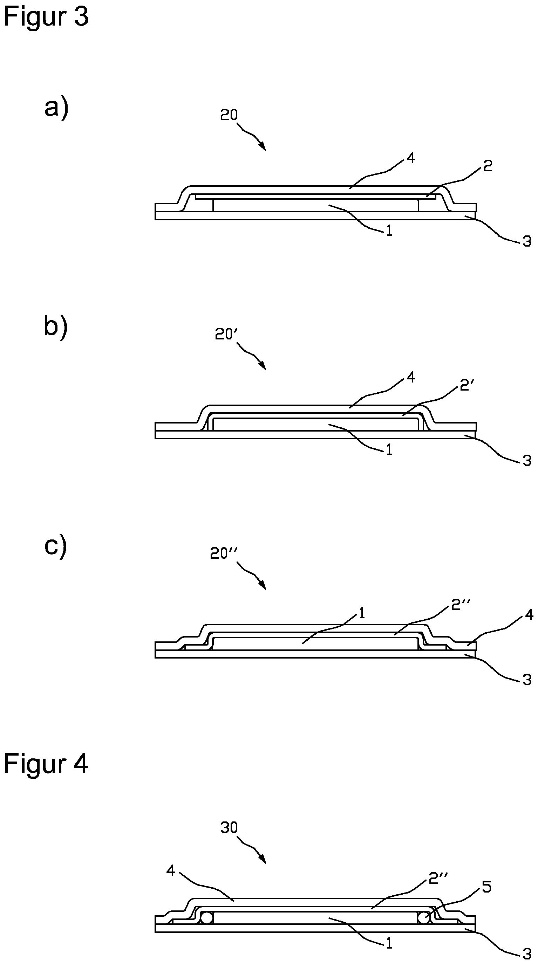

Die Querschnittsdarstellungen der Figur 3 veranschaulichen verschiedene Ausgestaltungen einer zweiten Ausführungsform eines transdermalen Applikationssystems mit einer Begrenzung des Austritts von Matrix und/oder Wirkstoff aus dem von einer wirkstoffundurchlässigen Rückschicht abgedeckten Bereich. Im Gegensatz zur oben beschriebenen ersten Ausführungsform überdeckt bei dieser zweiten Ausführungsform eine Deckschicht 4 die Bereiche der Schutzfolie 3 außerhalb des Verbunds aus Rückschicht 2 und Matrix 1, wodurch der Verbund innerhalb von Deckschicht 4 und Schutzfolie 3 eingeschlossen ist. Die auch als Overtape bezeichnete Deckschicht 4 dient dem zuverlässigen großflächigen Anbringen des TDS auf der Haut. In Bezug auf den Verbund aus Matrix und Rückschicht entsprechen die Ausgestaltungen der Darstellungen a, b und c von Figur 3 den jeweiligen Ausgestaltungen von Figur 1, bzw. Figur 2a oder Figur 2b.

Bei der in Darstellung a von Figur 3 vorgestellten Ausgestaltung 20 legt sich die Rückschicht 2 nicht an die Seitenränder der Matrix 1 an. Ein Kontaktieren der Matrixränder durch die Deckschicht 4 wird durch den Überstand der Rückschicht 2 dennoch wirksam verhindert. Bei genügend groß gewähltem Überstand kann sich auch eine kalt fließende Matrix 1 nicht so stark verformen, dass sie in Kontakt mit der Deckschicht 4 gerät. Vorteilhaft wird für diese Ausführungsform eine Rückschicht mit einer Dicke im Bereich von 50 bis 500 µm und/oder ein Material mit einer Steifigkeit gewählt, das einer Verformung durch die Deckschicht entgegenwirken kann.

Bei Matrices, die einen hohen kalten Fluss aufweisen, wird dagegen die in Darstellung b gezeigte Ausgestaltung 20' bevorzugt, bei der die überlappende Rückschicht an den Seitenflächen der Matrix 1 anliegt und hierüber einem Ausfließen der Matrix und/oder einem Austritt von Wirkstoff aus dem von der Rückschicht 2 umgrenzten Bereich wirksam vorbeugt. Um einem Kriechen der Wirkstoffe und/oder der Matrix über die Oberfläche der abziehbaren Schutzfolie wirksam zu begegnen, wird der Überlapp der Rückschicht 2, wie in Darstellung c von Figur 3 anhand der Ausgestaltung 20" veranschaulicht ist, vorzugsweise so gestaltet, dass er mit seinem äußeren Rand flächig auf der Oberfläche der Schutzfolie 3 aufliegt.

Eine dritte Ausführungsform 30 eines transdermalen Applikationssystems mit einer Begrenzung des Austritts von Matrix und/oder Wirkstoff aus dem von einer wirkstoffundurchlässigen Rückschicht abgedeckten Bereich ist mittels einer schematischen Querschnittsdarstellung in Figur 4 veranschaulicht. Diese Ausführungsform unterscheidet sich von der Ausgestaltung 20" der zweiten Ausführungsform nach Darstellung c von Figur 3 durch eine Randstütze 5, die die Matrixränder umschließt. Die Randstütze 5 stabilisiert die Matrix und gewährleistet insbesondere bei stark kaltfließenden Matrices, dass sich die Auflagefläche der Matrix auf der Schutzfolie bzw. bei Anwendung des TDS auf der Haut nicht vergrößert. Die Querschnittsgeometrie der Randstütze 5 kann wie im dargestellten Beispiel kreisförmig sein, sie kann jedoch auch, z.B. im an die Matrix 1 angrenzenden Bereich, plane Abschnitte aufweisen. Die Querschnittsgeometrie der Randstütze 5 kann ferner so ausgebildet sein, dass sie einen faltenfreien Verlauf des Rückschichtüberstands von der Oberkante der Matrixränder auf die Schutzfolie sicherstellt. Selbstverständlich kann die dritte Ausführungsform 30 auch ohne Deckschicht 4 ausgeführt sein.

Die oben beschriebenen Ausführungsformen können auf verschiedene Weisen hergestellt werden, wobei dem Fachmann die einzelnen Verfahrensschritte wie Beschichten, Laminieren und Vereinzeln bekannt sind. Für die Herstellung von Matrixpflastern mit mehrschichtigem Aufbau kann beispielsweise auf die Druckschrift EP 1 062 926 verwiesen werden.

Zur Herstellung von transdermalen Systemen gemäß der zweiten und dritten Ausführungsform werden bevorzugt die nachfolgend beschriebenen Verfahren angewandt.

Gemäß einem ersten Verfahren wird auf eine später die Rückschicht bildende Backingfolie zunächst eine Schablone aufgebracht. Die Schablone weist an den Stellen Öffnungen auf, an denen die Matrix auf der Backingfolie angeordnet sein soll. Anschließend wird das wirkstoffhaltige Matrixmaterial gleichmäßig auf die Schablonenseite des Verbunds aus Backingfolie und Schablone aufgebracht, so dass die Backingfolie durch die Öffnungen der Schablone hindurch mit dem Matrixmaterial selektiv beschichtet wird. Die Beschichtung kann mit bekannten Verfahren, wie z.B. mit einem Messergießer erfolgen. Nach dem anschließenden Abziehen der Schablone verbleiben an den Stellen der Backingfolie Inseln aus Matrixmaterial, an denen sich zuvor die Öffnungen der Schablone befanden.

In einem nachfolgenden Schritt wird die mit Inseln aus Matrixmaterial versehene Backingfolie ganzflächig mit einer abziehbaren Schutzfolie kaschiert, so dass die Schutzfolie alle Matrixinseln mit dem erforderlichen Überlapp bedeckt. Als Schutzfolie wird vorzugsweise eine Releaselinerfolie verwendet, die zumindest an der mit der Matrix in Kontakt tretenden Oberfläche siliconisiert ist. In einem nächsten Schritt wird die Backingfolie so gestanzt, dass Rückschichtinseln entstehen, von denen jede jeweils eine Matrixinsel mit einem wie oben beschriebenen Überlapp bedeckt. Diese solcherart mit einer Rückschicht bedeckten Matrixinseln werden nachfolgend als TDS-Kerne bezeichnet. Nach dem Abziehen des nicht als Rückschicht dienenden Teils der ausgestanzten Backingfolie wird die mit den TDS-Kernen belegte Releaselinerfolie mit einer haftklebenden Overtapefolie kaschiert. Die Overtapefolie deckt dabei die TDS-Kerne ab und legt sich zwischen diesen an die Oberfläche der Schutzfolie an. Die einzelnen transdermalen Applikationssysteme werden anschließend aus dem Folienverbund ausgestanzt. Außer den beschriebenen Schritten kann das Verfahren natürlich auch weitere Schritte aufweisen, die zur Herstellung eines bestimmten TDS erforderlich sind, beispielsweise einen Schritt zum Trocknen der aufgetragenen Matrix.

Bei der Herstellung transdermaler Applikationssysteme gemäß der ersten Ausführungsform entfällt der Schritt zum Aufbringen der Overtapefolie. Ferner kann die Backingfolie, falls nichtselbstklebende Matrices verwendet werden oder falls die Rückschicht auch zum Fixieren des TDS auf der Haut verwendet werden soll, vor dem Aufbringen des Matrixmaterials mit einer Kleberschicht beschichtet werden. Das Auftragen der Kleberschicht kann dabei gemustert erfolgen, beispielsweise unter Verwendung einer Schablone oder einer Siebdruckvorrichtung.

Zur Herstellung von transdermalen Applikationssystemen gemäß der dritten Ausführungsform werden die Randstützen 5 vor dem Aufbringen der Releaselinerfolie um die Matrixinseln gelegt.

Bei einem zweiten Herstellungsverfahren wird das Matrixmaterial zunächst auf eine Trägerfolie aufgetragen auf der das Matrixmaterial zwar haftet, die aber von der Matrixschicht im weiteren Verlauf des Verfahrens wieder abgezogen werden kann. Als Trägerfolie wird vorzugsweise eine beschichtete Folie, z.B. eine Releaselinerfolie verwendet. Die Matrixbeschichtung wird in einem nachfolgenden Schritt mit einer abziehbaren Schutzfolie kaschiert, die eine größere Haftung auf dem Matrixmaterial aufweist als die Trägerfolie. Geeignete Materialien für die Schutzfolie und die Trägerfolie sind beispielsweise Polyester, Polypropylen, Polyvinylchlorid, Aluminium und Papier, wobei zumindest eine Seite der Schutzfolie oder Trägerfolie eine Siliconbeschichtung, Polyethylenbeschichtung, Fluorsiliconbeschichtung oder Polytetrafluorethylenbeschichtung aufweist. In darauffolgenden Schritten wird der Schichtteilverbund aus Trägerfolie und Matrixbeschichtung zur Ausbildung der Matrixkerne gestanzt und das Stanzgitter, d.h. der nicht die Matrixkerne umfassende Teil des Schichtteilverbunds aus Trägerfolie und Matrixbeschichtung abgezogen. Schließlich werden die auf den Matrixkernen verbliebenen Reste der Trägerfolie entfernt, beispielsweise mithilfe eines Saughebers, bevor die mit den Matrixkernen versehene abziehbare Schutzfolie mit einer Backingfolie kaschiert wird. In einem nachfolgenden Schritt wird die Backingfolie so gestanzt, dass wie oben beschriebene TDS-Kerne mit überlappender Rückschicht auf der Schutzfolie ausgebildet werden. Nach dem Entfernen des Backingfolienstanzgitters wird die nun mit den TDS-Kernen belegte Schutzfolie mit einer haftklebenden Overtapefolie wie im oben beschriebenen ersten Verfahren kaschiert, und anschließend werden die transdermalen Applikationssysteme schließlich durch Stanzen vereinzelt.

Durch die Erfindung ist es möglich, ein transdermales therapeutisches System zu schaffen, dass den effektiven Einschluss des bzw. der Wirkstoffe in der Matrix des Systems wie auch der Matrix selbst auch während längerer Lagerungszeiten ermöglicht.

Insbesondere ist es durch die Erfindung möglich, eine kaltfließende Matrix zur Bereitstellung eines transdermalen therapeutischen Systems zu verwenden, ohne dass dadurch die Lagerfähigkeit des gebrauchsfertigen Systems negativ beeinträchtigt würde. Vorteil der Verwendung einer kaltfließenden Matrix ist die häufig gute bis sehr gute Klebrigkeit der Matrix. Entsprechend betrifft die Erfindung in einer Ausführungsform die Verwendung einer kaltfließenden Matrix zur Herstellung eines transdermalen therapeutischen Systems, das sowohl lagerstabil ist als auch gute bis sehr gute Klebeigenschaften aufweist.

## Patentansprüche

1. Transdermales Applikationssystem aufweisend:
eine kaltfließende wirkstoffhaltige Matrix (1), wobei die die kaltfließende wirkstoffhaltige Matrix bildenden Polymere ausgewählt sind unter: natürlichem Kautschuk, synthetischem Kautschuk, Homo-Polymeren auf Acrylbasis, Polyvinylacetat, Polyisobutylen, Siliconen, Hydrogelen und Polyethylenoxid-Polymeren,
eine wirkstoffundurchlässige Rückschicht (2) und
eine abziehbare Schutzfolie (3),
wobei die Matrix (1) zwischen der Rückschicht (2) und der Schutzfolie (3) angeordnet ist und die Rückschicht (2) so ausgebildet ist, dass sie die Ränder der Matrix (1) überlappt, und
wobei der Überstand der Rückschicht (2) über die Ränder der Matrix (1) größer als der am Rand der Matrix (1) vorliegende Abstand zwischen Rückschicht (2) und abziehbarer Schutzfolie (3) ausgeführt ist,
wobei der Überstand der Ränder der Rückschicht (2) über die Ränder der Matrix (1) einen Wert aufweist, der aus dem Bereich des Zweifachen bis Zehnfachen des Abstands der Rückschicht (2) von der Schutzfolie (3) am Rand der Matrix (1), vorzugsweise aus dem Bereich des Zweifachen bis Fünffachen und besonders bevorzugt aus dem Bereich des Zweifachen bis Dreifachen ausgewählt ist, und
wobei zumindest die auf der Schutzfolie (3) aufliegende Fläche der Rückschicht (2) mit einer haftklebenden Beschichtung versehen ist.

2. Transdermales Applikationssystem nach Anspruch 1, das ferner eine Deckschicht (4) aufweist, wobei die Deckschicht die Rückschicht (2) vollständig überdeckt und mit der abziehbaren Schutzfolie (3) außerhalb der Ränder der Rückschicht (2) haftklebend verbunden ist.

3. Transdermales Applikationssystem nach Anspruch 2, worin die Deckschicht (4) haftklebend ausgeführt ist.

4. Transdermales Applikationssystem nach einem der Ansprüche 2 oder 3, worin der Überstand der Deckschicht (4) über die Ränder der Rückschicht (2) wenigstens das Dreifache des Abstands zwischen Deckschicht und abziehbarer Schutzfolie (3) am Rand der Matrix (1) beträgt.

5. Transdermales Applikationssystem nach einem der vorhergehenden Ansprüche, das ferner eine Matrix-Randumfassung (5) aufweist, die an die Ränder der Matrix angrenzt und von der wirkstoffundurchlässigen Rückschicht (2) überdeckt ist.

6. Transdermales Applikationssystem nach einem der vorhergehenden Ansprüche, bei dem die in der wirkstoffhaltigen Matrix enthaltenen Wirkstoffe ausgewählt sind unter: Fentanyl und/oder Buprenorphin und/oder Remifentanil und/oder Sufentanil.

7. Verfahren zur Herstellung eines transdermalen Applikationssystems, das aufweist: eine kaltfließende wirkstoffhaltige Matrix (1), wobei die die kaltfließende wirkstoffhaltige Matrix bildenden Polymere ausgewählt sind unter: natürlichem Kautschuk, synthetischem Kautschuk, Homo-Polymeren auf Acrylbasis, Polyvinylacetat, Polyisobutylen, Siliconen, Hydrogelen und Polyethylenoxid-Polymeren, eine wirkstoffundurchlässige Rückschicht (2) und eine abziehbare Schutzfolie (3), mit Schritten zum
Versehen zumindest der zum Aufliegen auf der Schutzfolie (3) vorgesehenen Fläche der Rückschicht (2) mit einer haftklebenden Beschichtung,
Ausbilden der wirkstoffundurchlässigen Rückschicht (2) so, dass die Rückschicht (2) die Ränder der wirkstoffhaltigen Matrix (1) im gebrauchsfertigen transdermalen Applikationssystem überlappt, und so, dass der Überstand der Rückschicht (2) über die Ränder der Matrix (1) größer als der am Rand der Matrix (1) vorliegende Abstand zwischen Rückschicht (2) und abziehbarer Schutzfolie (3) ist, wobei der Überstand der Ränder der Rückschicht (2) über die Ränder der Matrix (1) einen Wert aufweist, der aus dem Bereich des Zweifachen bis Zehnfachen des Abstands der Rückschicht (2) von der Schutzfolie (3) am Rand der Matrix (1), vorzugsweise aus dem Bereich des Zweifachen bis Fünffachen und besonders bevorzugt aus dem Bereich des Zweifachen bis Dreifachen ausgewählt ist.

8. Verfahren gemäß Anspruch 7, das ferner einen Schritt zum Auswählen des Wirkstoffs aus: Fentanyl und/oder Buprenorphin und/oder Remifentanil und/oder Sufentanil umfasst.

## Claims

1. A transdermal application system, comprising:
a cold flowing active-substance-containing matrix (1), wherein the polymers forming the cold flowing active-substance-containing matrix are selected from: natural rubber, synthetic rubber, acrylic-based homopolymers, polyvinyl acetate, polyisobutylene, silicones, hydrogels and polyethylene oxide polymers,
a backing layer (2) impermeable to active substances and
a releasable protective film (3),
wherein the matrix (1) is arranged between the backing layer (2) and the protective film (3), the backing layer (2) is configured to overlap the edges of the matrix (1) with the overlap of the backing layer (2) over the edges of the matrix (1) being larger than the distance between the backing layer (2) and the releasable protective film (3) at the edge of the matrix (1),
wherein the overlap of the edges of the backing layer (2) over the edges of the matrix (1) has a value selected from the range of two to ten times the distance of the backing layer (2) from the protective film (3) at the edge of the matrix (1), preferably from the range of two to five times, and particularly preferably from the range of two to three times, and
wherein at least the surface of the backing layer (2) resting on the protective film (3) is provided with a pressure-sensitive adhesive coating.

2. The transdermal application system according to claim 1, further comprising a cover layer (4), the cover layer covering the backing layer (2) completely and being pressure-sensitively bonded to the releasable protective film (3) outside the edges of the backing layer (2).

3. The transdermal application system according to claim 2, wherein the cover layer (4) is configured to be pressure-sensitive.

4. The transdermal application system according to claim 2 or 3, wherein the overlap of the cover layer (4) over the edges of the backing layer (2) is at least three times the distance between the cover layer and the releasable protective film (3) at the edge of the matrix (1).

5. The transdermal application system according to one of the preceding claims, further comprising a matrix edge surround (5) adjacent to the edges of the matrix and covered by the active-substance-impermeable backing layer (2).

6. The transdermal application system according to one of the preceding claims, wherein the active substances contained in the active-substance-containing matrix are selected from: fentanyl and/or buprenorphine and/or remifentanil and/or sufentanil.

7. A method for preparing a transdermal application system comprising: a cold flowing active-substance-containing matrix (1) with the polymers forming the cold flowing active-substance-containing matrix being selected from: natural rubber, synthetic rubber, acrylic-based homopolymers, polyvinyl acetate, polyisobutylene, silicones, hydrogels and polyethylene oxide polymers; an active-substance-impermeable backing layer (2); and a releasable protective film (3), the method comprising steps for
providing at least the surface of the backing layer (2) intended to rest on the protective film (3) with a pressure-sensitive adhesive coating,
forming the active-substance-impermeable backing layer (2) such that the backing layer (2) overlaps the edges of the active substance-containing matrix (1) in the ready-to-use transdermal application system, and such that the overlap of the backing layer (2) over the edges of the matrix (1) is larger than the distance between the backing layer (2) and the releasable protective film (3) at the edge of the matrix (1),wherein the overlap of the edges of the backing layer (2) over the edges of the matrix (1) has value selected from the range of two to ten times the distance of the backing layer (2) from the protective film (3) at the edge of the matrix (1), preferably from the range of two to five times, and particularly preferably from the range of two to three times.

8. The method according to claim 7, further comprising a step for selecting the active substance from: fentanyl and/or buprenorphine and/or remifentanil and/or sufentanil.

## Revendications

1. Système d'application transdermique présentant :
une matrice (1) à fluage à froid contenant un agent actif, où les polymères formant la matrice (1) à fluage à froid contenant l'agent actif sont sélectionnés parmi : le caoutchouc naturel, le caoutchouc synthétique, des homo-polymères à base acrylique, le poly(acétate de vinyle), le polyisobutylène, les silicones, les hydrogels et les polymères de poly(oxide d' ethylène),
une couche arrière (2) imperméable à l'agent actif,
un film protecteur (3) détachable,
où la matrice (1) est agencée entre la couche arrière (2) et le film protecteur (3) et la couche arrière (2) est formée de façon à chevaucher les bords de la matrice (1) et où le surplomb de la couche arrière (2) au-delà des bords de la matrice (1) est réalisé plus grand que la distance présente au bord de la matrice (1) entre la couche arrière (2) et le film protecteur (3) détachable,
où le surplomb des bords de la couche arrière (2) au-delà des bords de la matrice (1) présente une valeur sélectionnée parmi le domaine de deux fois à dix fois, de préférence parmi le domaine de deux fois à cinq fois, très préférentiellement parmi le domaine de deux fois à trois fois la distance de la couche arrière (2) au film protecteur (3) au bord de la matrice (1), et
où du moins la surface de la couche arrière (2) reposant sur le film protecteur (3) est pourvue d'un revêtement adhésif.

2. Système d'application transdermique selon la revendication 1, présentant en outre une couche de revêtement (4), où la couche de revêtement recouvre intégralement la couche arrière (2) et est attachée de façon adhésive avec le film protecteur (3) détachable en dehors des bords de la couche arrière (2).

3. Système d'application transdermique selon la revendication 2, dans lequel la couche de revêtement (4) est réalisée de façon adhésive.

4. Système d'application transdermique selon la revendication 2 ou 3, dans lequel le surplomb de la couche de revêtement (4) au-delà des bords de la couche arrière (2) est au moins trois fois la distance entre la couche de revêtement et le film protecteur (3) détachable au bord de la matrice (1).

5. Système d'application transdermique selon une des revendications précédentes, présentant en outre un entourage de bord de matrice (5) juxtaposé aux bords de la matrice et qui est recouvert par la couche arrière (2) imperméable à l'agent actif.

6. Système d'application transdermique selon une des revendications précédentes, dans lequel les agents actifs contenus dans la matrice contenant l'agent actif sont sélectionnés parmi : le fentanyl et / ou la buprenorphine et / ou le rémifentanil et / ou le sufentanil.

7. Procédé pour la fabrication d'un système d'application transdermique, présentant : une matrice (1) à fluage à froid contenant l'agent actif, où les polymères formant la matrice (1) à fluage à froid contenant l'agent actif sont sélectionnés parmi : le caoutchouc naturel, le caoutchouc synthétique, des homo-polymères à base acrylique, le poly(acétate de vinyle), le polyisobutylène, les silicones, les hydrogels et les polymères de poly(oxide d'ethylène), une couche arrière (2) imperméable à l'agent actif et un film protecteur (3) détachable, avec des étapes pour
pourvoir au moins la surface de la couche arrière (2) prévue à reposer sur le film protecteur (3) d'un revêtement adhésif,
former la couche arrière (2) imperméable à l'agent actif de façon à ce que, dans le système d'application transdermique prêt à l'emploi, la couche arrière (2) chevauche les bords de la matrice (1) contenant l'agent actif, et de façon à ce que le surplomb de la couche arrière (2) au-delà des bords de la matrice (1) soit plus grand que la distance présente au bord de la matrice (1) entre la couche arrière (2) et le film protecteur (3) détachable, où le surplomb des bords de la couche arrière (2) au-delà des bords de la matrice (1) présente une valeur sélectionnée parmi le domaine de deux fois à dix fois, de préférence parmi le domaine de deux fois à cinq fois, très préférentiellement parmi le domaine de deux fois à trois fois la distance de la couche arrière (2) au film protecteur (3) au bord de la matrice (1).

8. Procédé selon la revendication 7, comprenant en outre une étape de sélection de l'agent actif parmi : le fentanyl et / ou la buprenorphine et / ou le rémifentanil et / ou le sufentanil.
